# EUROPEAN PATENT APPLICATION

(11) **EP 3 434 289 A1**
(43) Date of publication of application: **30.01.2019**
(21) Application number: 17460045.2
(22) Date of filing: 26.07.2017
(51) Int. Cl.: A61L 2/08, A61L 2/10, A61L 2/232, F21V 23/04

(54) **SWITCHING SELF-SANITIZING DEVICE**

(71) Applicant: ABB Schweiz AG, 5400 Baden (CH)
(72) Inventor: Rybak, Andrzej, 30-732 Krakow (PL); Kasza, Krzysztof, 30-716 Krakow (PL); Porebska, Renata, 32-600 Oswiecim (PL); Sekula, Robert, 30-613 Krakow (PL)
(74) Representative: Chochorowska-Winiarska, Krystyna

(57) **Abstract**

The present invention relates to a switching self-sanitizing device which can be used in the environment where the users touch the device in order to change the status for open to close or vice versa. A switching self-sanitizing device comprising switching element (2) with an antibacterial coating (3) having an antibacterial agent in the structure of the coating (3), where the agent is activated by illumination of the coating (3) with an UV light source. The switching self-sanitizing device comprises a control box (8) mounted on the base plate (1) in which a microprocessor (13) and a converter (12) electrically connected with themselves are placed. The converter (12) is electrically connected with a proximity sensor (7) placed outside the box (8) on the front side of the plate (1). The proximity sensor (7) is electrically connected with the microprocessor (8) to which a switch element (14) is connected for closing or opening an illumination circuit which is powered through an internal plug (11) placed in a connection box (9) mounted on the plate (1). In the connection box (9) an external plug (10) is inserted for powering the microprocessor (8), the proximity sensor (7) and a the UV light source through the converter (12).

## Description

The present invention relates to a switching self-sanitizing device which can be used in the environment where the users touch the device in order to change the status for open to close or vice versa. The invention is useful at the locations where probability of bacterial infection and their spreading and deposition on various surfaces is very high, especially for such applications as a low voltage equipment in hospitals, where proposed solution allows more efficient sanitizing of a housing of an electrical devise.

Many electrical devices as switches, sockets, touch panels, keyboards are often used in the environment where probability of bacterial infection is high. The hospitals, health centers can be a source of bacteria due to frequent touching by the infected people. Such a circumstances enhance risk of the uncontrolled bacteria growth and expansion of the infection. In order to prevent bacterial infection, there are many disinfection methods on the market, both physical e.g. heat, UV and gamma radiation, and chemical e.g. alcohols, oxidants, aldehydes.
The housings of electrical devices like light switches, that are placed in the environment with need of extra protection against microbes, are mainly made of polymer materials. Such devices are touched by many persons and thus particularly exposed to accumulation and growth of the bacteria. For that reason there is a need for method, that would assure self-sanitizing of these devices. The common solution to that problem is regular cleaning of the touched surface, covering that surface with antibacterial materials, or incorporation of antibacterial additives into the bulk of the material used for housing. However, standard methods, like manual cleaning of surface of electrical devices with use of chemical disinfectants, are not performing effectively for example because of too long intervals between each cleaning, and they are also not cost effective (e.g. manpower and material cost).
Alternatively, UV (ultraviolet) radiation is applied. It is crucial to exterminate bacteria effectively and in a short time. The antibacterial properties of the material or its surface might be reduced or lost with time. The UV radiation source might be malfunctioning or break down. The ultraviolet (UV) radiation is known to have ability to exterminate microorganisms, such as various kinds of bacteria. It is often applied in various sanitizing devices. The antibacterial properties of titanium dioxide Ti02 are also known. Moreover, they can be highly enhanced by ultraviolet activation of that material by means of UV-induced Ti02 photocatalytic process. The standard photocatalysis mechanism goes as following: a semiconductor is irradiated with energy of light equal or greater than its band gap. Energy of UV-light is then absorbed what results in excitation of electrons from the valence band to the conduction band. This leads to the formation of electron-hole pairs which can migrate to the catalyst surface and participate in redox reactions. The produced reactive oxygen species cause lethal injury to microbes. There are numerous compounds which have been examined as possible semiconductor photocatalysts, e.g. metal oxides, like Ti02, ZnO, Zr02, V205, Fe203, Sn02. However, the most widespread photocatalyst for use in antibacterial applications is titanium dioxide, which is a wide band semiconductor (3.2 eV) and thus needs the UV excitation. Bearing in mind cost, chemical and photochemical stability, availability, and absence of toxicity, the most appropriate catalyst is Ti02.

From US patent application US 2007/0258852 there is known a passenger interface device which comprises an input member having a contact surface that is adapted to be touch by an individual and a radiation - activated disinfectant coating on the contact surface. A source of radiation irradiates the disinfectant to disinfect the contact. The disinfectant comprises a photocatalist in the form of as titanium dioxide Ti02. The source of radiation comprises an ultra violet light source or a laser. The method depicted in the application US 2007/0258852 has crucial disadvantages, namely constant UV irradiation and use of UV transparent material which can be hazardous to humans. Taking into account drawbacks of standard disinfection methods and also disadvantages of more developed solutions proposed in the patent application US 2007/0258852 it would be beneficial to deliver an electrical switching device which will allow the automatically controlled and safe disinfection process by means of a smart digital technology.

A essence of the invention is that a switching self-sanitizing device comprises a control box mounted on the base plate in which a microprocessor and a converter electrically connected with themselves are placed, and the converter is electrically connected with a proximity sensor placed outside the box on the front side of the plate. The proximity sensor is electrically connected with the microprocessor to which a switch element is connected for closing or opening an illumination circuit which is powered through an internal plug placed in a connection box mounted on the plate. In the connection box an external plug is inserted for powering the microprocessor, the proximity sensor and a the UV light source through the converter.

Preferably the UV light source is at least one UV emitting diode or an array of UV emitting diodes placed in at least one pair of containers, where each containers is situated opposite to each other.

Preferably the both containers form two casings for the UV light source in a way that at least one UV emitting diode is placed in at least one pair of the containers and the UV light emitted by a diode is directed directly on an external surface of the coating of the touching element..

Preferably the proximity sensor is an infrared photo-element or an ultrasound detector.

Preferably an antibacterial agent embedded in the coating is chosen as one being of titanium dioxide Ti02, zinc oxide ZnO, zirconium dioxide Zr02, vanadium oxide V2O5, iron oxide Fe2O3, tin dioxide Sn02 or their combination.

Preferably the coating is made of a thin film structure and has a thickness less than 100 µm and comprises up to 90 wt.% of antibacterial agent.

Preferably the touching element has an antibacterial agent embedded in its structure with concentration of 0.2 - 5.0 wt.%.

Preferably the microprocessor is adapted for counting the number of switching operations activated periodical by the switch element and activated by the proximity sensor in order to automatically regulate power and time of UV irradiation.

Preferably the microprocessor is adapted for turning UV emitting source in the states "ON" and "OFF" on the basis of the electric signal received from the proximity sensor.

The present invention in comparison with the prior art has many advantages. The titanium dioxide and ultraviolet radiation will be applied as two complementary disinfectant mechanisms in order to create effective all-in-one method for sanitizing a housing of an electrical device. In the present invention the UV radiation can be activated in a controlled manner for a short period of time or activated after the housing was touched by the user. The invention allows for controlling the UV irradiation, especially for switching off the UV when user is approaching what is very important from security and health point of view, as uncontrolled UV irradiation may cause eye damage. The invention allows to avoid excessive skin exposure and possible skin burns. Additionally, an automated regulation of UV irradiation has economic benefits as a power is not wasted for undesired and not necessary disinfection.

The present invention is presented in the exemplary embodiment in the drawing where:

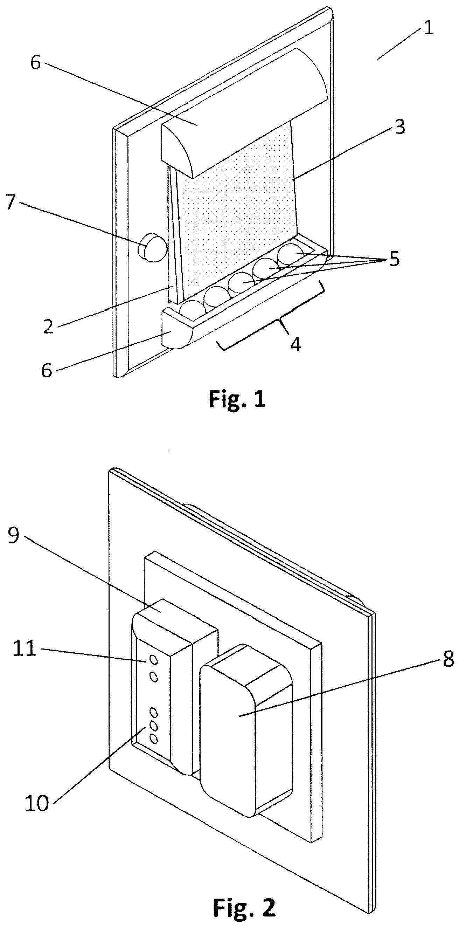

Fig 1 shows front of the switch in an axonometric view,
Fig 2 shows back of the switch in an axonometric view,

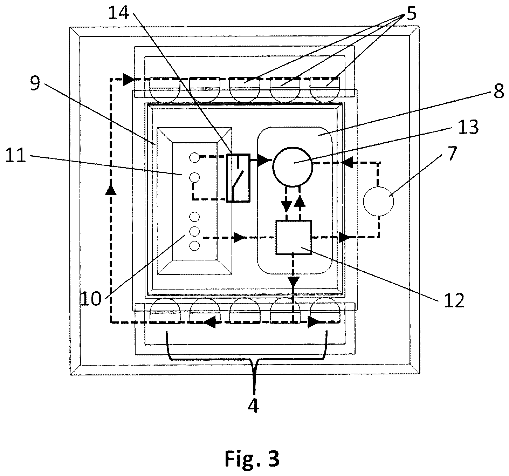

Fig 3 shows the back of the switch with a schematic diagram of connection between internal elements of the switch.

The switch comprises the base plate 1 on which a switching touching element 2 is mechanically mounted. The touching element is placed on the front side of the base plate 1 and has a form of a rocker (push pad) for touching by a user during the switching operation. The touching element 2 has an external surface 2a which is covered with an antibacterial coating 3 having a structure in which antibacterial agents are embedded. The agents are activated by the UV light. As the antibacterial agent in the antibacterial coating 3 the following compounds can be used as a single ingredient or their mixture: titanium dioxide Ti02, zinc oxide ZnO, zirconium dioxide Zr02, vanadium oxide V205, iron oxide Fe203, tin dioxide Sn02. These compounds can be applied by chemical vapor deposition, acoustic cavitation or other processes, suitable for direct fabrication of the antibacterial coating at the external surface 2a. The touching element 2 can be of any solid material, including polymers. The coating 3 consisting of antibacterial compounds can be also made of antibacterial agent mixed with binders or adhesives. Such created antibacterial coating 3 should be less than 100 µm thick and containing up to 90 wt.% of antibacterial agent. Alternatively, the touching element 2 can be also antibacterial by itself by incorporation of the antibacterial agent in the bulk of the material creating the touching element 2, for example by mixing of antibacterial agent with polymer during injection molding process and fabrication of touching element 2. The concentration of agent should be in this case at the level of 0.2 - 5.0 wt.%. Both methods of antibacterial agents addition, namely application of the antibacterial coating 3 and incorporation of antibacterial material into the bulk of the touching element 2, may be present together in order to increase the antibacterial effect. The UV light is generated by an array 4 of UV emitting diodes 5 connected in series. The UV light can be also emitted by a single diode what is not presented in the drawing. The emitting diodes 5 are placed in two containers 6 mounted to the plate 1. The first container 6a has a shape closing to the half of the gutter closed on the ends where the circular part of the gutter is protruding from the plate 1 and is placed on the plate 1 below the touching element 2. An open side of the gutter is directed upward. The second container 6b has a shape closing to the half of the gutter closed on the ends where the circular part of the gutter is protruding from the plate 1 and is placed on the plate 1 below the touching element 2. An open side of the gutter is directed downward. The both containers 6a and 6b form the two casings for the UV light source in a way that diodes 5 are partially enclosed in the container 6 and directs the UV light directly to an external surface of the coating 3 of the touching element 2. The container 6 prevents direct exposure of the user and neighboring environment to the UV light. The front side of the base plate 1 of the switch is equipped with proximity sensor 7 for detecting a close presence of the user. The proximity sensor can be an UV photoelement or an ultrasound detector. The sensor is placed outside the touching element 2 in such position that is easy visible to the user. On the back side of plate 1 there is placed a control box 8 having means for controlling the UV light emission. Also on the back side of plate 1 there is placed a connection box 9 having two kind of plugs, first internal plug 10 for connecting a main power supply, not presented in the drawing, and the second external plug 11 for connecting an electrical path, indicated in the drawing by a dotted line. The plugs 10 and 11 are equipped with typical fastening elements such as screws clamps, that are not shown in drawing. The main power supply provides the energy for UV diodes 5, proximity sensor 7 and control unit 8. The means for controlling the UV light emission have a form of a power converter 12 electrically connected with the microprocessor 13. The energy provided from the main power supply through the plug 11 is directed to the power converter 12. The power converted 12 enclosed inside the control unit 8, distributes energy from the main power source to the UV diodes 5, proximity sensor 7 and microprocessor 13 enclosed in control unit 8. Proximity sensor 7 sends information about close presence of the user to microprocessor 8. The plug 11 is connected with a switching element 14 which is connected with the microprocessor 13. The microprocessor 13 is adapted for counting the number of switching operations in time. The microprocessor 8 controls the power converter 12 and is adapted for turning UV diodes 5 in the states "ON" and "OFF" on the basis of the signal from the proximity sensor 7 and an internal algorithm of the microprocessor 13 evaluates optimal irradiation time basing on the number of switching operations.

The switching operation is done by changing the position of the switching element 2. The user touches surface 3 of the switching element 2 in order to change its position. During that operation any microorganisms such as bacteria or viruses, that are present on the user's hand, might be deposited on the surface 3 of the switching element 2. In order to prevent growth of these microorganisms the surface 3 is covered with antibacterial agent, such as titanium dioxide. The efficiency of eliminating microorganisms from the surface 3 is increased by activation of the antibacterial agent with UV light. The UV light is emitted by single UV diode or an array of such diodes. The array 4 of UV light diodes 5 is partially enclosed in the container 6. The shapes of the container 6 allows to direct the UV light into the surface 3 and protect the user and neighboring environment from direct exposure of the UV light. In order to increase safety the emission of the UV light is controlled. The proximity sensor 7 detects the presence of the users in close distance to the switch. In case of such presence, it sends information to the microprocessor 13, that is enclosed in the controlling box 8. The controlling algorithm of the microprocessor 13 blocks UV light emission if proximity sensor 7 detects close presence of the user. The controlling algorithm of the microprocessor 13 also computes the time for UV light emission, that will ensure the most efficient operation. The duration of the emission of the UV light is evaluated basing of the number of switching operations, i.e. the more often the switch is used the longer emission time would be required in order to sanitize the surface 3.

## Claims

1. A switching self-sanitizing device comprising switching element (2) with an antibacterial coating (3) having an antibacterial agent in the structure of the coating (3), where the agent is activated by illumination of the coating (3) with an UV light source, **characterized in that** it comprises a control box (8) mounted on the base plate (1) in which a microprocessor (13) and a converter (12) electrically connected with themselves are placed, and the converter (12) is electrically connected with a proximity sensor (7) placed outside the box (8) on the front side of the plate (1); the proximity sensor (7) is electrically connected with the microprocessor (8) to which a switch element (14) is connected for closing or opening an illumination circuit which is powered through an internal plug (11) placed in a connection box (9) mounted on the plate (1); and in the connection box (9) an external plug (10) is inserted for powering the microprocessor (8), the proximity sensor (7) and a the UV light source through the converter (12).

2. A switching self-sanitizing device according to claim 1, **characterized in that** the UV light source is at least one UV emitting diode (5) or an array of UV emitting diodes (4) placed in at least one container (6a) and (6b) situated opposite to each other.

3. A switching self-sanitizing device according to claim 2, **characterized in that** the both containers (6a) and (6b) form two casings for the UV light source in a way that at least one UV emitting diode (5) is placed in at least one container (6a) or (6b) and the UV light emitted by a diode (5) is directed directly on an external surface of the coating (3) of the touching element (2).

4. A switching self-sanitizing device according to any previous claims, **characterized in that** the proximity sensor (7) is an infrared photo-element or an ultrasound detector.

5. A switching self-sanitizing device according to any previous claims, **characterized in that** an antibacterial agent embedded in the coating (3) is chosen as one being of titanium dioxide Ti02, zinc oxide ZnO, zirconium dioxide Zr02, vanadium oxide V205, iron oxide Fe203, tin dioxide Sn02 or their combination.

6. A switching self-sanitizing device according to claim 5, **characterized in that** the coating (3) is made of a thin film structure and has a thickness less than 100 µm and comprises up to 90 wt.% of antibacterial agent.

7. A switching self-sanitizing device according to claim 5, **characterized in that** the touching element (2) has an antibacterial agent embedded in its structure with concentration of 0.2 - 5.0 wt.%.

8. A switching self-sanitizing device according to claim 1, **characterized in that** the microprocessor (13) is adapted for counting the number of switching operations activated periodical by the switch element (14) and activated by the proximity sensor (7) in order to automatically regulate power and time of UV irradiation.

9. A switching self-sanitizing device according to claim 1, **characterized in that** the microprocessor (8) is adapted for turning UV emitting source in the states "ON" and "OFF" on the basis of the electric signal received from the proximity sensor (7).
